# EUROPÄISCHE PATENTSCHRIFT

(11) **EP 0 708 325 B1**
(45) Veröffentlichungstag und Bekanntmachung des Hinweises auf die Patenterteilung: **28.04.1999**
(21) Anmeldenummer: 95111983.3
(22) Anmeldetag: 31.07.1995
(51) Int. Cl.: G01N 21/90

(54) **Verfahren und Vorrichtung zur Inspektion von Gegenständen, insbesondere von Flaschen**
Method and device for the inspection of objects, especially bottles
Méthode et dispositif d'inspection d'objets notamment de bouteilles

(30) Priorität: 20.10.1994 CH 3151/94
(43) Veröffentlichungstag der Anmeldung: 24.04.1996
(73) Patentinhaber: ELPATRONIC AG, CH-6303 Zug (CH)
(72) Erfinder: Buchmann, Christa, CH-8903 Birmensdorf (CH); Burri, Karl-Georg, CH-8942 Oberrieden (CH)

(56) Entgegenhaltungen:
- EP-A- 0 047 936
- EP-A- 0 371 546
- US-A- 5 256 871

## Beschreibung

Die Erfindung betrifft ein Verfahren gemäss Oberbegriff des Anspruchs 1. Ferner betrifft die Erfindung eine Vorrichtung zur Inspektion von Gegenständen gemäss Oberbegriff des Anspruchs 7.

Ein solches Verfahren ist z.B. aus US-A-5 256 871 bekannt, bei welchem mittels seitlich an einer Fördereinrichtung angeordneter Spiegel gleichzeitig zwei Bilder eines Gegenstandes erzeugt und von einer Kamera aufgenommen werden. Ein Verfahren ist aus der EP-A-209 077 (Kirin Beer KK) bekannt, bei welchem der Mündungsbereich von Flaschen mittels einer trichterförmigen Spiegelanordnung und einer CCD-Kamera inspiziert wird. Es entsteht dabei eine runde Abbildung mit konzentrischen Bereichen. Diese bekannte Anordnung hat den Nachteil, dass durch die eine runde Abbildung das rechteckige CCD-Element der Kamera schlecht ausgenützt wird. Ferner ist es natürlich möglich, den zu inspizierenden Bereich derart ganz zu erfassen dass mehrere Kameras in verschiedenen Winkelabständen um den Bereich herum angeordnet werden. Dies hat aber den Nachteil hoher Kosten, insbesonders da für die Betrachtung rasch bewegter Gegenstände - wie dies bei der Flascheninspektion der Fall ist - recht teure Kameras zum Einsatz gelangen müssen, und es hat andererseits den Nachteil, dass zur Weiterverarbeitung mehrere Bilder verschiedener Kameras vorliegen, was auch auf der Bildverarbeitungsseite den Aufwand wesentlich erhöht.

Der Erfindung liegt deshalb die Aufgabe zugrunde, ein Verfahren zu schaffen, welches solche Nachteile nicht aufweist.

Dies wird bei einem Verfahren der eingangs genannten Art durch die kennzeichnenden Merkmale des Anspruchs 1 erreicht.

Dadurch, dass auf dem Bildaufnahmeelement sechs Bilder des zu inspizierenden Gegenstandes erzeugt werden, kann das Bildaufnahmeelement optimal ausgenützt werden. Da eine Zahl von sechs Bildern auf dem einen Bildaufnahmeelement erzeugt wird bzw. mit einer Kamera aufgenommen wird, wird der apparative Aufwand vermindert. Es werden sechs Bilder aus sechs verschiedenen Blickwinkeln von einer Kamera aufgenommen. Die einzelnen Bilder können auf dem Bildaufnahmeelement nahe beieinander angeordnet werden und es muss nur ein Kameraausgang zur elektronischen Erfassung aller Bilder verarbeitet werden.

Die sechs Abbildungen werden durch mehrfache Spiegelung erzeugt. Die mehrfache Spiegelung bietet Vorteile. Es kann eine grosse Tiefenschärfe durch einen langen optischen Weg und den Einsatz eines Kameraobjektivs langer Brennweite erzielt werden. Es kann ein geringes Bauvolumen der Inspektionsvorrichtung durch Faltung des optischen Weges erzielt werden.

Bevorzugterweise erfolgt die Spiegelung mit planen Spiegeln. Dies hat den Vorteil der Vermeidung optischer Fehler, die bei der Verwendung gekrümmter Spiegel auftreten.

Weiter bevorzugt ist es, wenn die Strahlengänge zur Erzeugung aller Bilder gleich lang sind. Damit entstehen Bilder gleicher Grösse, was die Auswertung vereinfacht.

Weiter bevorzugt ist es, wenn die Anzahl Bilder durch die Spiegelungen in rechteckförmiger Anordnung erzeugt wird. Dies ergibt eine optimale Ausnützung des in der Regel rechteckigen Bildaufnahmeelementes.

Der Erfindung liegt ferner die Aufgabe zugrunde, eine Vorrichtung zu schaffen, welche die bereits vorstehend genannten Nachteile der bekannten Vorrichtungen nicht aufweist.

Dies wird bei einer Vorrichtung der eingangs genannten Art durch die kennzeichnenden Merkmale des Anspruchs 7 erreicht.

Im folgenden werden Ausführungsbeispiele der Erfindung anhand der Zeichnungen näher erläutert. Dabei zeigt
Figur 1 grob schematisch eine bekannte Möglichkeit zur Inspektion eines Gegenstandes;
Figur 2 den Gegenstand von Figur 1 von oben mit den gewünschten Ansichtsrichtungen;
Figur 3 grob schematisch eine Vorrichtung zur Durchführung der Erfindung;
Figur 4 ebenfalls schematisch die Anordnung der Bilder auf einem Bildaufnahmeelement;
Figur 5 eine schematisch dargestellte Spiegelanordnung von oben bzw. von der Kamera her gesehen;
Figur 6 die in Figur 5 gezeigte Spiegelanordnung in der einen Seitenansicht;
Figur 7 die in Figur 5 gezeigte Spiegelanordnung in der anderen Seitenansicht;
Figur 8 schematisch eine Ausführungsform mit zusätzlichen Spiegeln und einem Linsensystem;
Figur 9 die um 90° gedrehte Darstellung der Ausführung nach Figur 8; und
Figur 10 die Anordnung einer zusätzlichen Abbildung auf dem CCD-Element.

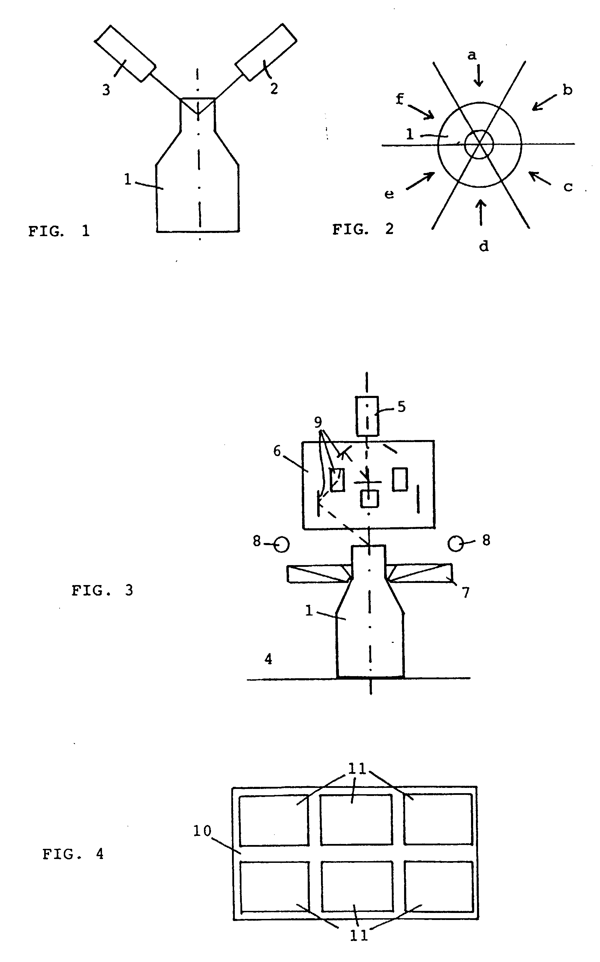

Die Figuren 1 und 2 zeigen einen zu inspizierenden Gegenstand 1 von der Seite bzw. von oben. Bei dem gezeigten Gegenstand handelt es sich um einen Behälter, z.B. eine PET-Mehrwegflasche, welche beim Mehrwegrücklauf inspiziert wird. Dies ist indes nur als Beispiel zu verstehen, da es sich bei dem Gegenstand 1 um einen beliebigen Gegenstand handeln könnte. Bei der in den Figuren 1 und 2 gezeigten PET-Flasche soll z.B. der Mündungsbereich inspiziert werden. Es könnten dazu nach Stand der Technik mehrere Kameras 2, 3 usw. eingesetzt werden, welche den Mündungsbereich von verschiedenen Seiten aufnehmen. Die aufgenommenen Bilder werden dann in einer nicht dargestellten Bildverarbeitungseinrichtung analysiert und es wird festgestellt, ob der Behälter 1 im Mündungsbereich einen Defekt aufweist, der dessen Ausscheidung aus dem Mehrwegumlauf bedingt. Um eine genügende Inspektionsgenauigkeit zu erreichen, muss der gesamte Mündungsumfang betrachtet werden. Wie in Figur 2 ersichtlich, können dazu z.B. sechs Ansichten a bis f, welche um jeweils 60° verschoben sind, aufgenommen werden und analysiert werden. Es ist ersichtlich, dass auf die dargestellte Weise dazu sechs Kameras notwendig sind und die einzelnen Bilder der sechs Kameras verarbeitet werden müssen. Dies ist sehr aufwendig, da die Flaschen mit hoher Geschwindigkeit (z.B. mit 750 Flaschen/Minute) durch die Inspektionseinrichtung laufen, was entsprechend teure Kameras und Bilderfassungseinrichtungen bedingt.

Die Erfindung schlägt daher einen anderen Weg ein. Figur 3 zeigt ebenfalls schematisch einen Behälter 1, wiederum eine PET-Mehrwegflasche, die zu inspizieren ist. Zu diesem Zweck ist nun eine einzige Kamera 5 vorgesehen, welche mehrere Abbilder der Flaschenmündung über eine Abbildungseinrichtung 6 aufnimmt. Die Abbildungseinrichtung 6 ist dabei so ausgestaltet, dass mehrere verschiedene Abbildungen der Flaschenmündung auf dem Bildaufnahmeelement der Kamera 5 erzeugt werden. Figur 4 zeigt schematisch ein solches Bildaufnahmeelement 10, wie es z.B. in der Kamera 5 vorhanden sein kann. In der Regel handelt es sich dabei um ein rechteckiges CCD-Bildaufnahmeelement. Durch die Abbildungseinrichtung 6 werden nun sechs verschiedene Abbildungen 11 des Mündungsbereichs der Flasche 1 erzeugt und gemeinsam auf das CCD-Element 10 gegeben. Dabei werden die sechs verschiedenen Bilder 11 in einer der CCD-Form angepassten Anordnung, also z.B. in der gezeigten rechteckigen Anordnung auf dem CCD-Element erzeugt. Die sechs Bilder 11 können dabei z.B. die verschiedenen Bilder a bis f gemäss Figur 2 sein. Durch die Abbildungseinrichtung 6 werden also gleichzeitig die sechs Bilder auf das Kameraobjektiv bzw. auf das Bildaufnahmeelement 10 abgebildet, so dass dieses Bildaufnahmeelement optimal ausgenützt wird. Die sechs verschiedenen Ansichten des Mündungsbereichs liegen dann auch als ein einziges Kameraausgangssignal vor, so dass auch nur eine Weiterverarbeitungseinheit für das Kamerasignal vorgesehen sein muss und nicht mehrere solche Einheiten, wie dies bei der herkömmlichen Ausführung nach Figur 1 der Fall gewesen ist.

Die Abbildungseinrichtung 6 enthält eine Mehrzahl von vorzugsweise planen Spiegeln, welche es der Kamera 5 erlauben, gleichzeitig mehrere verschiedene Ansichten des Gegenstandes zu betrachten. Es sind dies sechs Ansichten, wie bereits geschildert worden ist. Bei der dargestellten Inspektion von PET-Flaschen ist vorzugsweise unter der Mündung eine Reflexionsfläche 7 angeordnet, welche das Licht einer Beleuchtungseinrichtung 8 in der Regel diffus reflektiert, um den Mündungsbereich zu beleuchten. Auch die Reflexionsfläche 7, welche auch eine Leuchtfläche mit integrierter Beleuchtung sein könnte, ist vorzugsweise an die erzeugte Anzahl der Bilder so angepasst, dass die Fläche 7 aus mehreren aneinandergrenzenden Flächen aufgebaut ist, die der Anzahl Abbildungen entspricht. Dadurch ergibt sich eine Konzentration des Lichtes jeweils auf den entsprechenden abzubildenden Mündungsbereich. Bei der Inspektion von PET-Flaschen befindet sich die Flasche 1 in einer Inspektionseinrichtung und wird von einer nicht näher dargestellten Fördereinrichtung 4 an der feststehenden Abbildungseinrichtung 6 und Kamera 5 vorbeigeführt. Die Fördereinrichtung, welche z.B. als Karussellfördereinrichtung ausgestaltet sein kann wird hier, da bekannt, nicht weiter erläutert.

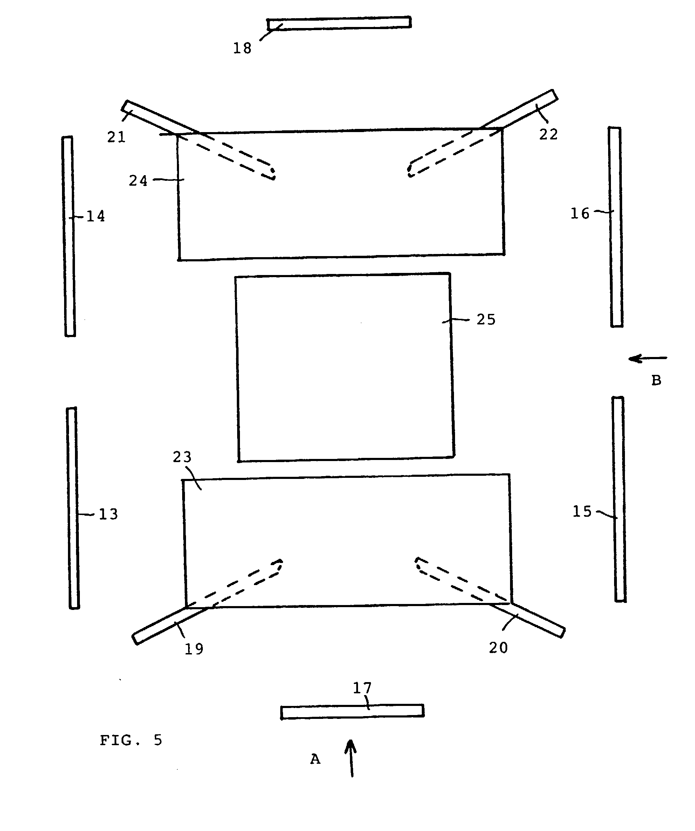

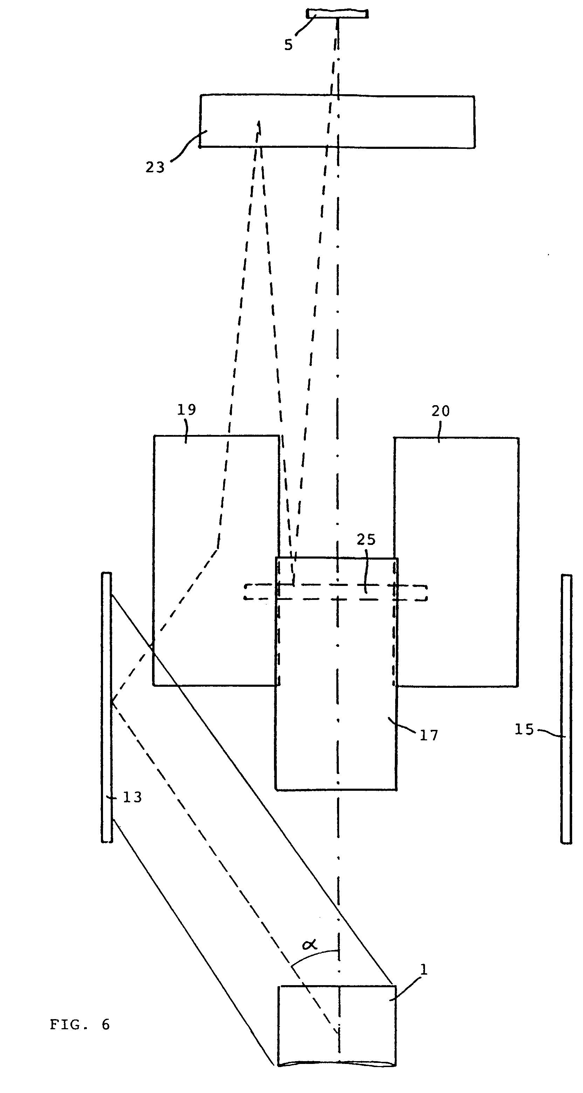

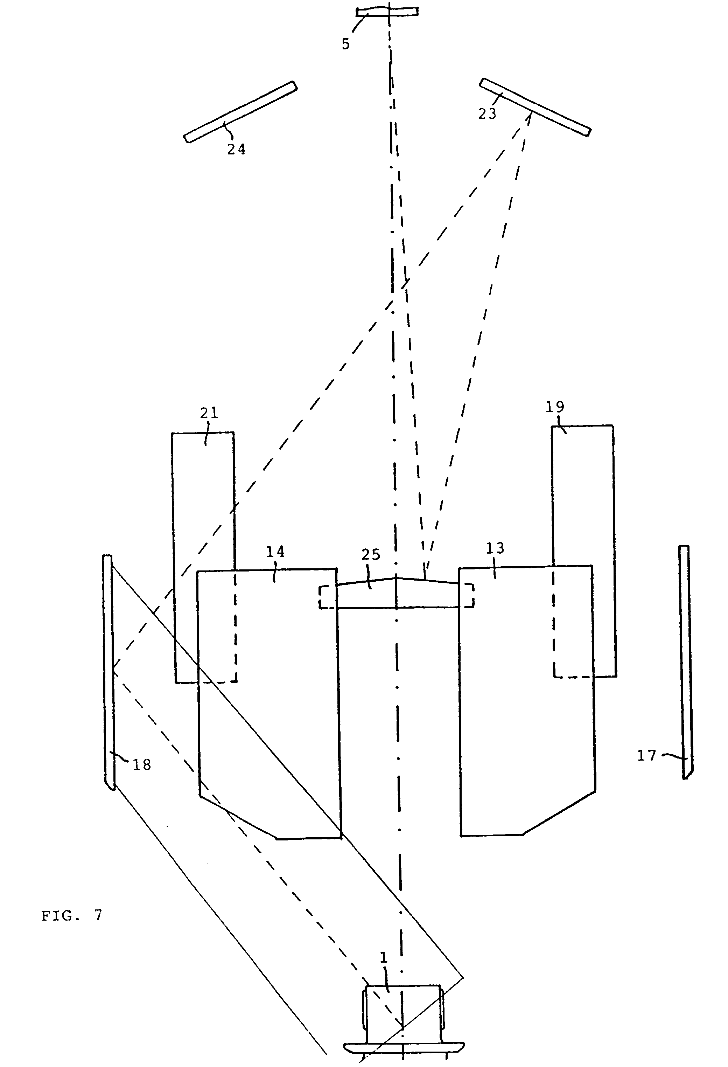

Im folgenden wird ein Beispiel einer Abbildungseinrichtung 6 anhand der Figuren 5, 6 und 7 näher erläutert, wobei hier ebenfalls, wie bereits dargestellt, gleichzeitig sechs Abbildungen des Mündungsbereichs erzeugt werden. In den Figuren 5 bis 6 sind zur Vereinfachung der Figuren die Spiegel nur schematisch als spiegelnde Flächen dargestellt und die entsprechenden Halterungen und das die Spiegel umgebende Gehäuse sind nicht dargestellt. Figur 5 zeigt eine erste Ansicht der Abbildungseinrichtung von oben her, d.h. von der Kameraseite her. Die nachfolgenden Abbildungen 6 und 7 zeigen dieselbe Abbildungseinrichtung aus seitlicher Sicht und zwar in Figur 6 von der Seite her gemäss dem Pfeil A in Figur 5 und in Figur 7 von der Seite her gemäss dem Pfeil B in Figur 5. Die Abbildungseinrichtung ist mit planen Spiegeln aufgebaut, was den bereits eingangs genannten Vorteil aufweist, dass keine Abbildungsfehler entstehen, die bei der Verwendung von gekrümmten Spiegeln unvermeidlich sind.

Die Abbildungsvorrichtung weist im gezeigten Beispiel einen dachförmigen Spiegel 25 auf, welcher jeweils den letzten Spiegel vor dem Objektiv 5 bildet, welches in den Figuren 6 und 7 ersichtlich ist. Der jeweils erste Spiegel für die jeweilige Abbildung wird von den Spiegeln 13, 14, 15, 16, 17 und 18 gebildet, entsprechend den sechs gewünschten Ansichten. Die getrennt dargestellten Spiegel 13 und 14 bzw. 15 und 16 könnten auch als ein durchgehender Spiegel ausgeführt sein, was bei der tatsächlichen Ausführung die Halterung erleichtern würde.

In Figur 6 ist der Strahlengang für eine der Ansichten des Mündungsbereichs des Behälters 1 dargestellt. Dabei verläuft der Strahlengang zunächst via den Spiegel 13 über den Spiegel 19 auf den Spiegel 23 und von diesem zurück auf den Spiegel 25 und von dort zum Objektiv 5. Derselbe Strahlengang gilt sinngemäss auch für die anderen drei Ansichten, welche jeweils über den Erstspiegel 14 bzw. 15 bzw. 16 gewonnen werden. Figur 7 zeigt andererseits den Strahlengang, welcher für diejenigen beiden Ansichten des Mündungsbereichs des Behälters 1 gilt, welcher über die beiden ersten Spiegel 17 bzw. 18 erfolgt, wobei nur der Strahlengang über den Spiegel 18 eingezeichnet ist. Dieser verläuft also über den Spiegel 18 auf den Spiegel 23 und von dort zurück zum Spiegel 25 und danach zum Objektiv 5. Der bevorzugte Winkel alpha zur senkrechten Längsachse des Behälters liegt im Bereich von 30° bis 50° und beträgt insbesondere ca. 38°.

Bei der gezeigten Abbildungseinrichtung sind alle sechs Strahlengänge im wesentlichen gleich lang, d.h. es entstehen sechs im wesentlichen gleich grosse Bilder auf dem Bildaufnahmeelement 10. Ferner sind bei dem gezeigten Beispiel die Planspiegel so angeordnet und auch in ihren Randbereichen so geformt, dass keine Abschattungen entstehen, d.h. die anderen Strahlengänge werden durch die Spiegel des jeweils einen Strahlenganges nicht gestört.

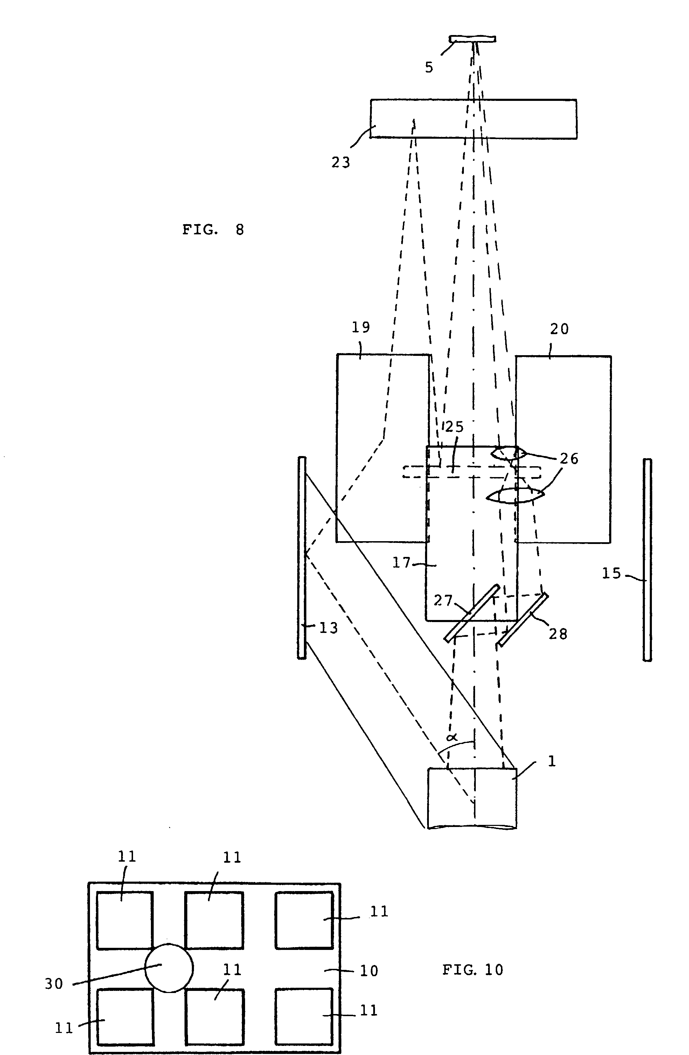

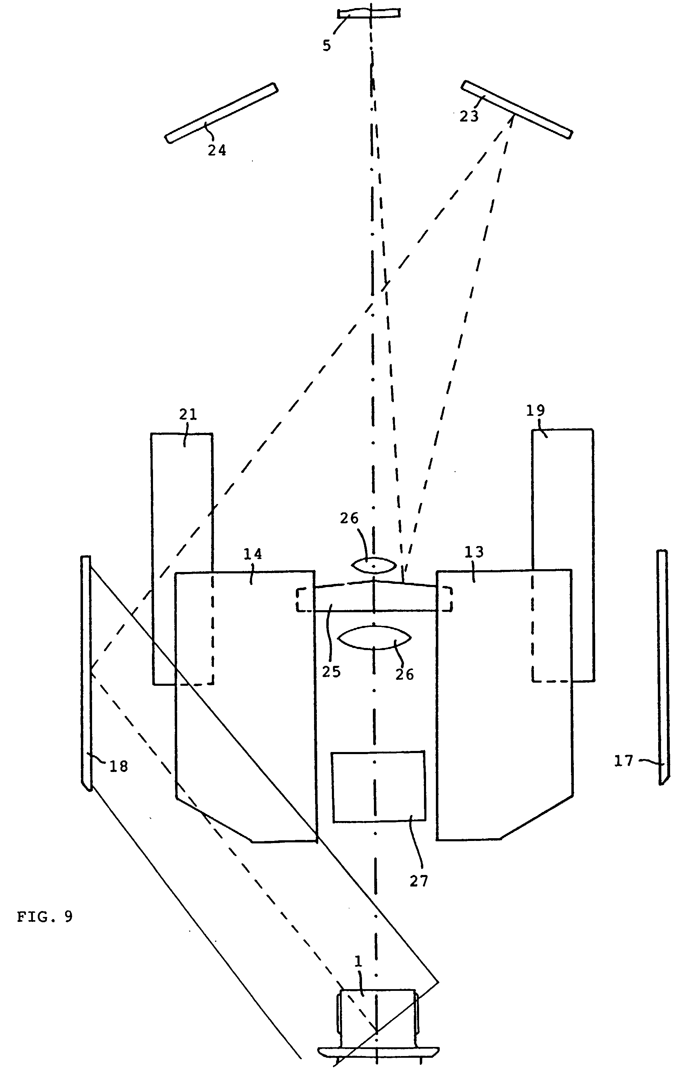

Figur 8 zeigt eine Ausführungsform der Abbildungseinrichtung entsprechend derjenigen der Figuren 5 bis 7 mit einem zusätzlichen Linsensystem 26 zur zusätzlichen Betrachtung des Gegenstandses von oben. Das Spiegelpaar 27,28 sorgt dabei für einen seitlichen Versatz der Abbildung. Figur 10 zeigt die entsprechende Anordnung auf dem CCD-Element 10 mit der zusätzlichen Abbildung 30. Das Linsensystem 26 dient zur Anpassung der Vergrösserung und der Schärfeebene. Es kann eine beliebige Anzahl Linsen aufweisen, und es kann Linsen enthalten, die die Ansicht in eine Richtung strecken (z.B. Zylinderlinsen), was eine bessere Ausnützung des CCD-Elementes ergibt. Figur 9 zeigt die Ausführung von Figur 9 um 90° gedreht.

Anstelle der Spiegel 27,28 kann der seitliche Versatz auch durch ein anderes optisches Element erfolgen, z.B. durch ein Prisma mit zwei verspiegelten Flächen, oder durch ein unverspiegeltes Prisma, in dem der Versatz durch Brechung erfolgt.

## Patentansprüche

1. Verfahren zur Inspektion von Gegenständen (1), insbesonders des Mündungsbereichs von Flaschen, bei welchem eine optische Abbildung auf ein Bildaufnahmeelement (10) einer Kamera (5) erfolgt und das Bild ausgewertet wird, dadurch gekennzeichnet, dass durch eine oberhalb des Gegenstandes angeordnete Spiegeleinrichtung sechs Abbildungen (11;30) des Gegenstandes auf dem Bildaufnahmeelement (10) erzeugt werden, welche zusammen eine 360° Ansicht des Gegenstandes bilden.

2. Verfahren nach Anspruch 1, dadurch gekennzeichnet, dass die Abbildungen (11) mittels planer Spiegel (9) erzeugt werden.

3. Verfahren nach einem der Ansprüche 1 oder 2, dadurch gekennzeichnet, dass der Strahlengang zur Erzeugung der verschiedenen Abbildungen (11) im wesentlichen für jede Abbildung gleich lang ist, so dass gleich grosse Bilder (11) erzeugt werden.

4. Verfahren nach einem der Ansprüche 2 oder 3, dadurch gekennzeichnet, dass für verschiedene Abbildungen (11) verschiedene Anzahlen von Spiegelungen erfolgen.

5. Verfahren nach einem der Ansprüche 1 bis 4, dadurch gekennzeichnet, dass als Bildaufnahmeelement ein rechteckiger oder quadratischer Sensor (10) vorgesehen ist und dass darauf die Abbildungen (11) in rechteckiger bzw. quadratischer Anordnung erzeugt werden.

6. Verfahren nach einem der Ansprüche 1 bis 5, dadurch gekennzeichnet, dass die Abbildungen in einem Winkel von ungefähr 30° bis 50°, insbesondere ca. 38° zur senkrechten Achse des Gegenstandes erfolgen.

7. Vorrichtung zur Inspektion von Gegenständen (1), insbesonders des Mündungsbereichs von Flaschen, bei welcher mittels einer Abbildungseinrichtung (6) eine optische Abbildung auf ein Bildaufnahmeelement (10) einer Kamera (5) erzeugt wird, und wobei die Abbildungseinrichtung mehrere plane Spiegel (9; 13-25) enthält und jede Abbildung durch mehrere Spiegelungen erzeugt, dadurch gekennzeichnet, dass die Abbildungseinrichtung oberhalb des Gegenstandes angeordnet und derart ausgestaltet ist, dass sechs verschiedene Abbildungen (11;30) des Gegenstandes auf dem einen Bildaufnahmeelement (10) erzeugbar sind, welche zusammen eine 360° Ansicht des Gegenstandes bilden.

8. Vorrichtung nach Anspruch 7, dadurch gekennzeichnet, dass verschiedene Abbildungen durch jeweils eine unterschiedliche Anzahl Spiegel erzeugt werden.

9. Vorrichtung nach Anspruch 8, dadurch gekennzeichnet, dass die Abbildungseinrichtung eine erste Gruppe von ersten Spiegeln (13,14,15,16) und eine zweite, näher zum Bildaufnahmeelement (10) hin angeordnete Gruppe von ersten Spiegeln (17,18), eine der ersten Gruppe zugeordnete dritte Gruppe von Zwischenspiegeln (19,20,21,22), und der ersten und der zweiten Gruppe zugeordnete Endspiegel (23,24,25) aufweist.

10. Vorrichtung nach einem der Ansprüche 7 bis 9, dadurch gekennzeichnet, dass zusätzlich eine Abbildung des Gegenstandes über eine Linsenanordnung (26) erfolgt.

11. Vorrichtung nach einem der Ansprüche 7 bis 10, dadurch gekennzeichnet, dass eine Beleuchtungseinrichtung (7,8) zur Beleuchtung des Gegenstandes vorgesehen ist, welche sechs aneinandergrenzende leuchtende Flächen aufweist.

## Claims

1. Process for the inspection of objects (1), in particular the mouth area of bottles, in which an optical image is made on an image recording element (10) of a camera (5) and the image is interpreted, characterized in that six images (11; 30) of the object, which combine to form a 360° view of the object, are generated on the image recording element (10) by a mirror system arranged above the object.

2. Process according to Claim 1, characterized in that the images (11) are generated by means of plane mirrors (9).

3. Process according to Claim 1 or Claim 2, characterized in that the ray path generating the various images (11) is of essentially equal length for each image so as to generate images (11) of equal size.

4. Process according to Claim 2 or Claim 3, characterized in that different series of reflections occur for different images (11).

5. Process according to any one of Claims 1 to 4, characterized in that a square or rectangular sensor (10) is provided as image recording element and in that the images (11) are generated thereon in a square or rectangular array.

6. Process according to any one of Claims 1 to 5, characterized in that the images are formed at an angle of about 30° to 50°, and in particular approx. 38°, to the vertical axis of the object.

7. Apparatus for the inspection of objects (1), in particular the mouth area of bottles, in which an optical image is generated by means of an imaging system (6) on an image recording element (10) of a camera (5), and in which the imaging system comprises a number of plane mirrors (9; 13-25) and each image is generated by multiple reflections, characterized in that the imaging system is arranged above the object and is constructed in such a way that six different images (11; 30) of the object, which combine to form a 360° view of the object, can be generated on the single image recording element (10).

8. Apparatus according to Claim 7, characterized in that different images are generated by different series of mirrors.

9. Apparatus according to Claim 8, characterized in that the imaging system comprises a first group of initial mirrors (13, 14, 15, 16) and a second group of initial mirrors (17, 18) located nearer to the image recording element (10), a third group of intermediate mirrors (19, 20, 21, 22) assigned to the first group, and final mirrors (23, 24, 25) assigned to the first and second groups.

10. Apparatus according to any one of Claims 7 to 9, characterized in that the object is additionally imaged via an arrangement of lenses (26).

11. Apparatus according to any one of Claims 7 to 10, characterized in that an illuminator device (7, 8) having six adjacent illuminating faces is provided to light the object.

## Revendications

1. Procédé d'inspection d'objets (1), notamment de la zone de l'embouchure de bouteilles, selon lequel une image optique est formée sur un élément d'enregistrement d'images (10) d'un appareil de prise de vues (5) et l'image est évaluée, caractérisé en ce qu'à l'aide d'un dispositif à miroirs disposé au-dessus de l'objet, on forme sur l'élément d'enregistrement d'image (10) six images (11;30) de l'objet, qui représentent conjointement une vue de l'objet sur 360°.

2. Procédé selon la revendication 1, caractérisé en ce que les images (11) sont formées au moyen de miroirs plans (9).

3. Procédé selon l'une des revendications 1 ou 2, caractérisé en ce que le trajet du faisceau servant à produire les différentes images (11) possède essentiellement la même longueur pour chaque image de sorte que des images (11) ayant des dimensions identiques sont produites.

4. Procédé selon l'une des revendications 2 ou 3, caractérisé en ce que des nombres différents de réflexions sont utilisés pour des images différentes (11).

5. Procédé selon l'une des revendications 1 à 4, caractérisé en ce qu'un capteur rectangulaire ou carré (10) est prévu comme élément d'enregistrement d'images et que les images (11) sont produites sur ce capteur avec une configuration rectangulaire ou carrée.

6. Procédé selon l'une des revendications 1 à 5, caractérisé en ce que les images sont formées sous un angle d'environ 30° à 50° et notamment d'environ 38° par rapport à l'axe vertical de l'objet.

7. Dispositif d'inspection d'objets (1), notamment de la zone d'embouchure de bouteilles, dans lequel une image optique est formée, au moyen d'un dispositif de formation d'images (6) sur un élément d'enregistrement d'images (10) d'un appareil de prise de vues (5), et dans lequel le dispositif de formation d'images contient plusieurs miroirs plans (9;13-25) et produit chaque image au moyen de plusieurs réflexions, caractérisé en ce que le dispositif de formation d'images est disposé au-dessus de l'objet et est agencé de telle sorte que six images différentes (11;30) de l'objet, qui forment conjointement une vue de l'objet sur 360°, sont produites sur un élément d'enregistrement d'images (10).

8. Dispositif selon la revendication 7, caractérisé en ce que différentes images sont produites par des nombres respectivement différents de miroirs.

9. Dispositif selon la revendication 8, caractérisé en ce que le dispositif de formation d'images comporte un premier groupe de premiers miroirs (13,14,15,16) et un second groupe de premiers miroirs (17,18), qui est disposé plus près de l'élément d'enregistrement d'image (10), un troisième groupe de miroirs intermédiaires (19,20,21,22) associé au premier groupe et des miroirs d'extrémité (23,24,25) associés aux premier et second groupes.

10. Dispositif selon l'une des revendications 7 à 9, caractérisé en ce qu'en outre une image de l'objet est formée par l'intermédiaire d'un dispositif à lentilles (26).

11. Dispositif selon l'une des revendications 7 à 10, caractérisé en ce qu'il est prévu un dispositif d'éclairement (7,8) servant à éclairer l'objet, qui comporte six surfaces éclairantes contiguës.
